# EUROPEAN PATENT SPECIFICATION

(11) **EP 4 326 202 B1**
(45) Date of publication and mention of the grant of the patent: **26.03.2025**
(21) Application number: 21723114.1
(22) Date of filing: 20.04.2021
(51) Int. Cl.: A61F 13/15, A61F 13/537, B32B 5/02, B32B 5/26, D01D 5/098, D04H 1/26, D04H 1/425, D04H 1/4374, D04H 1/56, D04H 1/60, D04H 13/00

(54) **LAMINATE FOR USE IN AN ABSORBENT ARTICLE**
LAMINAT ZUR VERWENDUNG IN EINEM SAUGFÄHIGEN ARTIKEL
STRATIFIÉ À UTILISER DANS UN ARTICLE ABSORBANT

(43) Date of publication of application: 28.02.2024
(73) Proprietor: The Procter & Gamble Company, Cincinnati, OH 45202 (US)
(72) Inventor: ERDEM, Gueltekin, Beijing 101312 (CN); LINDNER, Torsten, 65824 Schwalbach am Taunus (DE)
(74) Representative: P&G Patent Germany
(86) International application number: PCT/CN2021/088383
(87) International publication number: WO 2022/222029

(56) References cited:
- US-A1- 2005 148 261
- US-A1- 2017 021 589
- US-A1- 2019 247 238

## Description

### FIELD OF THE INVENTION

The invention relates to a laminate comprising a substrate layer and a fibrous layer. The laminate may in particular be used as an acquisition-distribution system in personal hygiene absorbent articles, such as diapers or feminine protections. The invention is also directed to a method for making the laminate, and an absorbent article comprising the laminate.

The laminate has excellent fluid transfer properties, integrity and is cost efficient to make. When used as an acquisition-distribution system, the laminate may be placed between the topsheet and the absorbent core of the absorbent article, such as a diaper, to more efficiently transfer a fluid such a urine from the topsheet into the absorbent core. The substrate layer may be a nonwoven acquisition layer and placed directly under the topsheet. The substrate layer may also comprise a hydrophilic treatment.

### BACKGROUND OF THE INVENTION

Personal hygiene absorbent articles such as baby diapers or adult incontinence products are multi-layered products typically comprising a topsheet on the wearer-facing side, a backsheet on the garment-facing side and an intermediate absorbent core comprising superabsorbent polymers. Most hygiene articles further comprise a nonwoven acquisition layer directly under the topsheet. Chemically cross-linked cellulose fibers have been further used as distribution layers ("patch") between an acquisition layer and the absorbent core in personal hygiene absorbent articles (see e.g. WO2008/155711, Hundorf et al.). These cross-linked fibers are laid down on the nonwoven acquisition layer during the diaper production process to form the patch. While these cross-linked fibers have excellent fluid distribution properties, the patch however lacks mechanical stability or "patch integrity" in use, which leads to tearing, crack formation and crunching which again impacts the liquid handling performance. This is ultimately resulting in higher leakage of the diaper, besides a negative (unappealing) appearance. Additionally, there are limitations in the in-process stabilization and fixation of cellulose patches (e.g. dust formation), as the fibers are weakly adhesively joined to adjacent substrates.

US2019/247,238A1, US2005/148,261A1 and US2017/021589A1 disclose a laminate comprising a substrate and a fibrous layer, wherein the fibrous layer is a coform layer comprising cellulose fibers bonded by meltblown fibers. These documents do not disclose the presence of irregularly shaped lumps of hotmelt material at least partially bonding the cellulose fibers together.

There is a need to develop a stable and coherent acquisition-distribution system for use in absorbent articles, overcoming shortcomings of previous approaches.

### SUMMARY OF THE INVENTION

The invention relates to a laminate comprising a substrate layer and a fibrous layer. The substrate layer and the fibrous layer each have an internal side and an external side. The substrate layer may typically be a fluid-permeable nonwoven. The substrate layer may be hydrophillically treated. The fibrous layer comprises cellulose fibers and a hotmelt material. The hotmelt material forms irregularly shaped lumps in the fibrous layer which at least partially bond together the cellulose fibers, thus forming a fibrous network providing integrity to the fibrous layer. The hotmelt material is at least distributed in a first thickness of the fibrous layer adjacent its internal side. The pores formed by the cellulose fibers are thus more resilient and kept open so that a fluid such as urine can easily transfer through at least this first thickness.

The fibrous layer may optionally comprise spunmelt (meltblown or spunbond) fibers intermixed with the cellulose fibers. The spunmelt fibers may be distributed through the thickness of the fibrous layer. Optionally, the fibrous layer may be further sprayed with a fiber coating composition on this external side to reduce dusting. Dusting refers to the undesired breakout or escape of cellulose fibers from a web that may contaminate a converting line such as a diaper line.

The laminate may be used as an acquisition-distribution system of an absorbent article such as a diaper. The acquisition-distribution system is disposed between the topsheet and the absorbent core. The acquisition-distribution system may be particularly used in combination with an absorbent core comprising no or only low amount of cellulose fibers as absorbent material in the absorbent core. The laminate may also be used for other applications, for example as a wipe for personal or surface cleaning. The invention is also directed to a method for continuously making the laminate. The laminate may be produced as a continuous web of laminate material that is formed into a roll for storage and transport to be converted with other layers in a converting line. The laminate may alternatively be produced directly on a manufacturing line and converted without storage and transport intermediate step into a finished article. The acquisition-distribution system can thus be made on-line, which means directly converted with other layers to make an absorbent article, but it can also be made off-line, which means produced at a different time and place than the absorbent article in which it will be integrated.

### BRIEF DESCRIPTION OF THE DRAWINGS

Fig. 1 is a schematic illustration of one way to manufacture of a laminate according to the invention;
Fig. 2 is a schematic cross-sectional view of a laminate according to the present invention, with the different constituents being shown separately, the substrate layer on top;
Fig. 3 is a cross-sectional SEM picture of a laminate according to the invention;
Fig. 4 and Fig. 5 are SEM pictures showing the irregularly shaped lumps punctually bonding the cellulose fibers;
Fig. 6 is a SEM picture of the external surface of the fibrous layer showing how the fibers are superficially coated with a hotmelt coating composition which has been sprayed on this surface;
Fig. 7 shows a schematical cross-section of an absorbent article incorporating the laminate of the invention as an acquisition-distribution system;
Fig. 8 illustrates an alternative to manufacture of a laminate according to the invention;
Fig. 9 illustrates an alternative to manufacture of a laminate according to the invention;
Fig. 10 illustrates an alternative to manufacture of a laminate according to the invention.

### Preamble

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present disclosure. The materials, methods, and examples disclosed herein are illustrative only and not intended to be limiting.

As used in the specification and in the claims, the term "comprising" may include the embodiments "consisting of and "consisting essentially of." The terms "comprise(s)," "include(s)," "having," "has," "can," "contain(s)," and variants thereof, as used herein, are intended to be openended transitional phrases, terms, or words that require the presence of the named ingredients/steps and permit the presence of other ingredients/steps. However, such description should be construed as also describing compositions or processes as "consisting of and "consisting essentially of the enumerated ingredients/steps, which allows the presence of only the named ingredients/steps, along with any impurities that might result therefrom, and excludes other ingredients/steps.

All % are by weight of the adhesive composition unless indicated otherwise.

### DETAILED DESCRIPTION OF THE INVENTION

### Laminate and Process for making

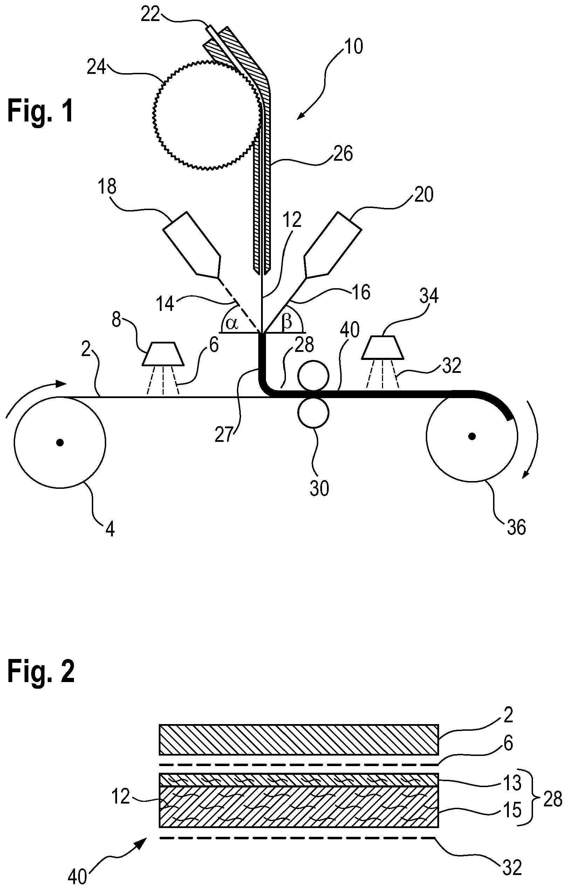

Applicant believes that the structure of the laminate of the invention will be better understood starting with the description of an exemplary process used to make the laminate. Fig. 1 describes schematically such a process for making a laminate according to the invention. As shown in Fig. 1, a substrate layer 2 is provided in a continuous manner, typically by unwinding a roll 4 of the substrate layer. The substrate layer is typically a nonwoven layer, for example an air-through bonded carded nonwoven. The substrate is advantageously hydrophilic, in particular if the substrate is later used as the acquisition layer in an acquisition-distribution system. Hydrophilic treatments such as a surface coating with a surfactant or other hydrophilic agent are typically made before the substrate layer is formed in the roll 4, as is known in the art. The substrate 2 may advantageously have a basis weight higher than 15 gsm to be able to provide sufficient permeability and void volume, for example between 20 gsm and 60 gsm. The nonwoven substrate may in particular comprise fibers that are at least 1.5 dpf (denier per filaments).

After unrolling, a first side of the substrate layer 2 may be applied with a conventional adhesive 6. This adhesive 6 may be sprayed from a glue spray nozzle 8 as the substrate layer is passed under the nozzle. The adhesive 6 may also be applied by coating stripes of adhesive using a contact applicator. This first side of the substrate layer will be referred herein as the internal side as it will face the fibrous layer in the laminate, and the opposite side of the substrate will be referred herein to as its external side. Likewise, the side of the fibrous layer 28 facing the substrate layer is referred herein as the internal side, and the opposite side of the fibrous layer is referred as the external side. The internal side of the substrate layer 2 thus faces the internal side of the fibrous layer 28 in the laminate 40.

The optional adhesive 6 deposited on the internal side of the substrate layer may comprise typical adhesive ingredient, in particular a polymer backbone, a tackifier and optional additives such as a plasticizer and antioxidants. The deposited adhesive basis weight can range from 1 to 15 g/m², in particular from 2 to 5 g/m². The function of the adhesive is to allow for a better integrity of the two layers of the laminate at their interface, but similar function may be provided by a mechanical or heat post-treatment, such as embossing, or sufficient integrity at the surface of the layer may be provided by the hotmelt material 14. Thus, while the adhesive treatment is advantageous, it is not always required.

The substrate layer 2 is then conveyed to the fibrous layer deposition station 10. The fibrous layer 28 is formed by intermixing cellulose fibers 12, a hotmelt material 14 and optionally spunmelt fibers 16. The deposition station 10 may be based on known stations used to make composite layer designed in the art as co-form or spinform. In the known spinform process, two streams of meltblown fibers are typically blown on each side of a central stream of cellulose fibers, where the fibers intermix, as disclosed for example in WO2020147231A1, WO2020/147232A1 (Xiamen Yanjan). In these known processes, the meltblown fibers are polypropylene fibers that are formed into very long and thin fibers (below 10 micrometer diameter).

In the present invention, a hotmelt material 14 is sprayed into the stream of cellulose fibers 12 instead of spinning long, thin polypropylene fibers by a meltblown process. Surprisingly, the inventors have found that even when using a meltblown die 18 (or both dies 18, 20), the hotmelt material 14 of the invention does not form long fine meltblown fibers, but instead forms irregularly shaped solidified droplets 17, referred herein as lumps. These lumps of hotmelt material adhere to and bond the cellulose fibers together. An interwoven network structure secures at least to some extent the cellulose staple fibers, increasing the integrity of the fibrous layer.

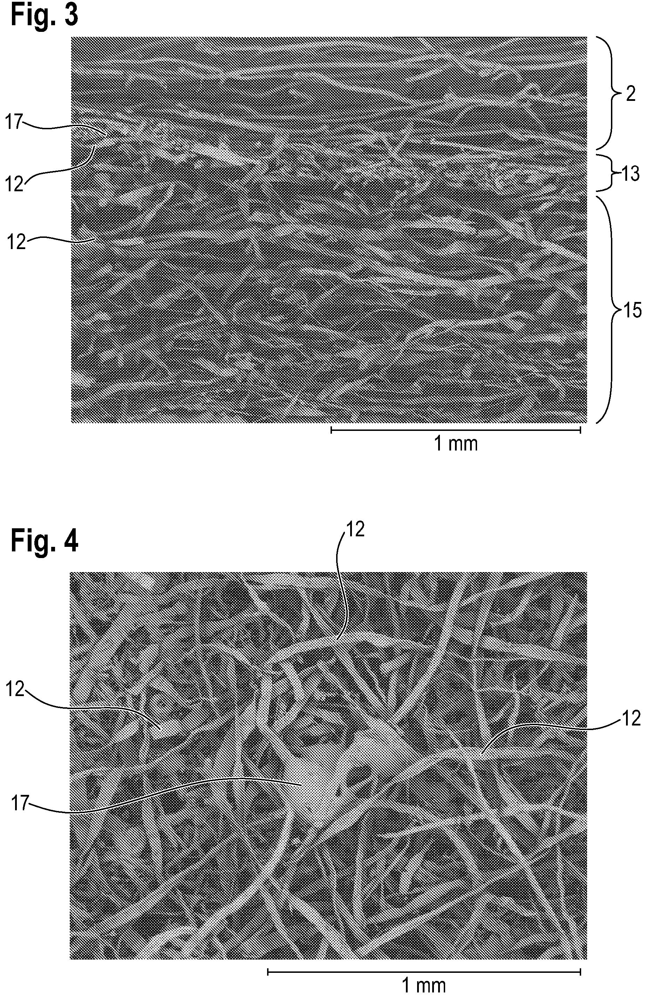

In the examples shown in Fig. 2 and Fig. 3, the lumps are present within and define a first thickness 13 of the fibrous layer 28 adjacent the internal side of the fibrous layer. However, this is not limiting, as the lumps 17 may be present over the whole thickness of the fibrous layer 28, or another portion thereof, as will be discussed further below in the section "other examples".

The cellulose fibers 12 may be typically obtained from wood pulp 22, which is loosened through the loosening roller 24 and passed through the spout 26 under the action of an auxiliary air flow to form a stream of cellulose fibers 12. Alternatively, or additionally, chemically cross-linked cellulose fibers may also be used. Chemically cross-linked cellulose fibers have been used as distribution layer material, as for example disclosed in US2013/331806 (Rosati et al.). PE powders may also be introduced in the cellulose fibers stream. Other fibers, such as synthetic fibers or other plant-origin fibers, may also be mixed with the cellulose fibers (including cross-linked cellulose fibers). It is also possible to mix superabsorbent particles with the cellulose fibers, as it is disclosed in WO2020/147232A1 (Yanjan) in the context of wipes, to provide absorbency properties to the layer. Alternatively, the fibrous layer and the laminate may be free of superabsorbent particles. The cellulose fibers 12, including any chemically cross-linked cellulose fibers, are however typically present in amount greater than 50% by weight of the total weight of the fibrous layer 28.

The deposition station 10 may optionally comprise a second meltblown die 20 disposed downstream of the stream 12 of cellulose fibers, so that the meltblown fibers are at least partially intermixed with the cellulose fibers on this second side of the fibrous layer. The deposition station may thus comprise two meltblown dies, one on each side of the cellulose chute. This is economical as such equipments are known and already used for the manufacture of co-form wipes. The hotmelt material 14 may also be sprayed into the stream of cellulose fibers 12 using other spraying equipment than a meltblown die, such as those dies used in spunbonding process or even an adhesive spray nozzle.

The fibrous layer 28 may thus optionally comprise meltblown fibers 16 made from a polymeric material, typically polypropylene, which is heated in the meltblowing process. The dissolved fine stream of thermoplastic resin is then ejected from the spinneret plates 20. The meltblown fibers are blown using a high temperature, high velocity hot gas stream into bundles of fibers having a fiber diameter < 10 µm. While meltblown fibers were used in the examples, spunbond fibers may also be introduced instead or in addition to the meltblown fibers 16. Spunmelt fibers are used herein means spunbond or meltblown fibers, as is known in the art. The projected hotmelt material 14 and if present the spunmelt fibers 16 (in particular meltblown fibers) are formed with a hot gas stream, and they each intersect one side of the cellulose pulp fibers stream 12 to form a combined fibrous stream 27 which deposits as a multilayer structural fibrous layer 28 on the substrate layer 2. The fibrous layer 28 may have a hotmelt material rich first side, and a meltblown fibers or spunbond fibers rich other side, with the cellulose fibers 12 extending through the whole thickness of the fibrous layer 28. The meltblown fibers 16, if present, typically consists of a single component having a high melting point such as polypropylene. Other fibers may also be included additionally or alternatively in the stream of cellulose fibers, such as bicomponent fibers or low melting polyester fibers.

The combined stream 27 of cellulose fibers, hotmelt material (and spunmelt fibers if present) is deposited on the internal side of the substrate layer 2 and forms there a unitary fibrous layer 28. The substrate layer 2 and the fibrous layer 28 may be partially attached by the adhesive 6 disposed at their interface.

In the fibrous layer 28, the weight percent of cellulose fibers 12 is typically greater than 50% of the total weight of the fibrous layer 28, typically between 65% and 80% of the total weight of the fibrous layer. The fibrous layer may comprise between 1% and 20% by weight of the hotmelt material forming the lumps 17. The fibrous layer may also comprise between 10% and 30% by weight of the spunmelt fibers (meltblown and/or spunmelt), when present.

Fig. 2 shows a schematic cross-sectional view of an exemplary laminate 40 according to the present invention. The substrate layer 2 is shown on top. The optional adhesive layer 6 is shown in dashed line between the substrate layer 2 and the fibrous layer 28. The fibrous layer 28 is preferably unitary, which means it cannot be mechanically separated in sub-layers without breaking its structure. As illustrated in Fig. 2, the fibrous layer 28 may comprise two main regions 13 and 15 in the thickness direction. The first region 13 corresponds to a first thickness of the fibrous layer adjacent the internal side of the fibrous layer. The hotmelt material is present and forms the lumps 17 that at least partially bond the cellulose fibers together in this first thickness 13. The second region 15 of the fibrous layer 28 is the rest of the fibrous layer that is substantially free of or does not comprise the hotmelt material lumps. The second region 15 thus essentially comprises cellulose fibers 12, optionally intermixed with spunmelt fibers, and/or other synthetic fibers or powders as mentioned above. Spunmelt (in particular meltblown) fibers may be present through the whole thickness of the fibrous layer 28, including the first region 13. The spunmelt fibers may be intermixed with the cellulose fibers throughout the whole thickness of the fibrous layer or only a portion of it. The lumps 17 of hotmelt material may be present in a first thickness 13 of the fibrous layer adjacent the internal side of the fibrous layer, as represented in Fig. 2, or in another thickness in the middle of the fibrous layer, or in another thickness adjacent the external side of the fibrous layer, or through the whole thickness of the fibrous layer.

The thickness of the first region 13 can be varied by changing the incident angles α of the hotmelt material spray relative to the stream of cellulose fibers 12 and the speed of the carrying medium. Likewise, the distribution of the spunmelt fibers may be varied by varying the incident angle β of the spunmelt die, relative to the stream of cellulose fibers 12, and/or varying the speed at which the spunmelt fibers is introduced in the stream 12. Of course, increasing the amount of hotmelt material or spunmelt fibers introduced also increase the penetration of these within the thickness of stream of cellulose fibers, as is known in the art.

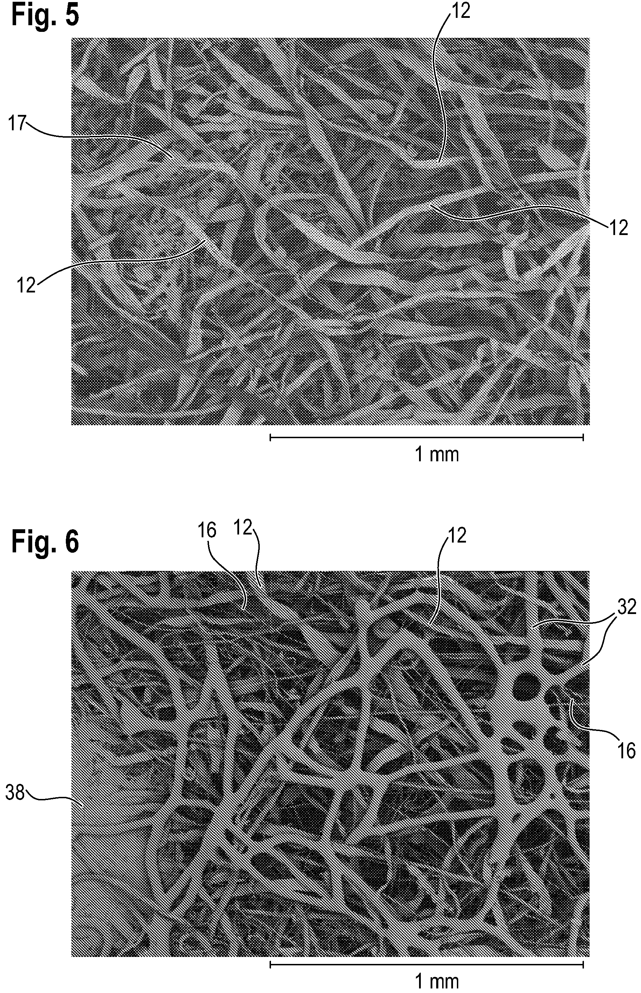

Fig. 3 shows a cross-sectional SEM picture of a laminate 40 as described in Fig. 2. A first thickness 13 of the fibrous layer comprises cellulose fibers 12, which have been stabilized by the hotmelt material 14. The hotmelt material form amorphous lumps 17 that adhere to and bond the cellulose fibers. In the example shown in Figs. 3-5, the hotmelt material consists of a single metallocene-catalyzed propylene-ethylene copolymer (Licocene^{®} 2502 ex Clariant). The first thickness 13 of the fibrous layer 28 where the hotmelt material is present may be less than 50% of the overall thickness of the fibrous layer, as the hotmelt material 14 quickly cools and forms the lumps 17 when it is sprayed into the flow of cellulose fibers. The hotmelt material thus typically does not penetrate through the whole thickness of the stream of cellulose fibers 12. However, it is not excluded that the first thickness 13 may also extend through more than half, up to the whole, of the thickness of the fibrous layer. This first region 13 of the fibrous layer is shown in the planar SEM pictures in Fig. 4 and Fig. 5, where the cellulose fibers 12 and the lumps 17 of hotmelt material 14 are visible.

Generally, the laminate may have a thickness in the range of from 0.5 to 5 mm as measured at a pressure of 0.5 kPa according to the Thickness Measurement method described herein. The thickness of the laminate may be in the range of from of 0.8 to 2.0 mm. The thickness of the substrate 2 may be measured separately and may make up at least 20%, preferably at least 30% of the total thickness of the laminate. Preferably, substrate 2 makes up at most 60% of the total laminate basis weight. The basis weight is calculated by dividing the weight of a sample by its area.

The fibrous layer 28 may further optionally comprise spunmelt fibers 16, in particular meltblown polypropylene fibers intermixed with the cellulose fibers. As schematically shown in Fig. 1, the spunmelt fibers may be typically introduced in the stream of cellulose fibers 12 on the side opposite the hotmelt material nozzle 18. The spunmelt fibers 16 are thus typically present at least in the second thickness 15 of the fibrous layer 28, but are optionally also present in the first thickness 13 of the fibrous layer.

The combined stream 27 is deposited on the substrate layer 2 where it forms the deposited fibrous layer 28. The laminate 28 may be further consolidated by passing the two layers between a pair of calendaring rolls 30 to provide for a more intimate contact between the layers. This can improve adhesion between the layers by the adhesive 6, if present. This calendaring step further improves the integrity of the laminate and reduce dust formation. At least one of the rolls 30 may be heated and optionally structured with protruding embossment features to form bonding points 38 by melting the spunmelt/meltblown fibers 16, if present, at the external surface of the fibrous layer. Total bonding area from such a bonding unit may range from 3% to 15% and preferably from 6% to 12% to enable integrity benefit without compressing the laminate excessively and affect fluid handling performance due to dense bond areas. Alternatively, the calendaring rolls 30 may be replaced by an oven to provide heat energy to melt any thermo-fusible fibers, in particular bicomponent fibers, present in the fibrous layer (see WO2020/147231A1 for disclosure of exemplary suitable thermofusible fibers).

In an optional step, the external surface of the fibrous layer 28 may be sprayed with a coating composition 32 that at least partially coats and binds the cellulose fibers and optional spunmelt fibers on this external side of the fibrous layer. The coating composition 32 may be a hotmelt composition that is sprayed using any conventional spray nozzle 34. Such a coating composition 32 is visible on the SEM picture of Fig. 6. Spraying a fiber coating composition at the external surface of the fibrous layer was found to advantageously to immobilize the cellulose and/or spunmelt fibers on this surface of the fibrous layer. These fibers may otherwise break off the fibrous layer and form "dust" that contaminates the manufacturing lines, in particular the absorbent article manufacturing line where the laminate may be incorporated as an acquisition-distribution system.

Any conventional fiber coating composition may be used, for example latexes. However, hotmelt compositions are preferred because they do not require a drying step unlike latexes. The hotmelt coating composition 32 may be the same or different than the hotmelt material 14 sprayed in the stream of cellulose fibers. In fact, the preferred hotmelt material discussed in more details below, were found to be particularly suitable as fiber coating composition. The hotmelt composition 32 and hotmelt material 14 may comprise the same polymer as backbone, in particular the same metallocene-catalyzed polymer, and even be identical, which simplifies inventory. The hotmelt coating composition may comprise hydrophilic melt additives, in particular those exemplified below.

### Hotmelt material 14

Any hotmelt materials that may be intermixed with the cellulose fibers and thereby forms the hotmelt material lumps 17 that at least partially bond the cellulose fibers together may be used. Suitable hotmelt materials however differ from high melting point polypropylenes that are conventionally used for meltblown fibers, as these polypropylenes form elongated very thin fibers when spun through the spinnerets (as illustrated by the meltblown fibers 16 on Fig. 6 for example).

Hotmelt material comprising a polymer backbone having a moderate chain length and molecular weight may thus be particularly suitable. The hotmelt material should also not require to be heated at a temperature that could damage the cellulose fibers for the selected processing application type (spunmelt, meltblown or spray). Hotmelt material having a viscosity at 170°C ranging from 100 mPa.s to 10,000 mPa.s are thus preferred.

While not limiting the scope of suitable hotmelt materials, it was found that metallocene-catalyzed polymers are particularly suitable to form the backbone of the hotmelt material. The hotmelt composition may typically comprise from 30% to 100% by weight of a metallocene-catalyzed propylene-based polymer (or mixture thereof), in particular from 50% to 100% by weight of one or more metallocene-catalyzed polymer(s). A single metallocene-catalyzed polymer may be used, or alternatively blends of metallocene-catalyzed polymers may be used. Thus, unless indicated otherwise, when using the term "a metallocene-catalyzed polymer" it is meant the "one or more metallocene-catalyzed polymer(s)".

The hotmelt material of the invention may in particular comprise one or more low molecular weight metallocene-catalyzed polymer having a peak molecular weight ranging from 10,000 g/mol to 130,000 g/mol. The peak molecular weight is measured according to the Peak Molecular Weight (Mp) Measurement Method disclosed further below.

Metallocene-catalyzed polymers typically have a regular spatial repeat monomer unit distribution and a narrow molecular weight distribution, as is known in the art. Propylene-based metallocene-catalyzed polymers may be preferably used. The propylene-based metallocene-catalyzed polymers may be homopolymers or copolymers, in particular propylene-ethylene copolymers. The propylene-ethylene copolymers comprise at least 50% by weight of the copolymer of propylene unit, in particular at least 60%, or at least 70%, or at least 80% by weight. The remaining monomers are ethylene monomers, and optionally other alpha olefin monomers may be present in the co-polymers, for example 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof. The exact monomer distribution is typically published by the supplier, but can also be determined by a suitable method, such as nuclear magnetic resonance or infrared spectroscopies.

Suitable metallocene-catalyzed propylene-ethylene copolymers are commercially available from Clariant under the trade name Licocene^{®}, with a broad range of properties such as molecular weight, viscosity, crystallinity, etc... US2016/053,149A1 assigned to Clariant also describes suitable co-polymers and on page 5 indicates that these examples were produced by the processes indicated in EP571,882. For a given catalyst system and given comonomer ratio, the molecular weight was regulated via the hydrogen partial pressure as molar mass regulator.

The hotmelt material may comprise a blend of two polymers having different molecular weight, in particular a low molecular weight metallocene-catalyzed propylene-ethylene copolymer having a peak molecular weight ranging from 10,000 g/mol to 130,000 g/mol, as indicated above, and a higher molecular weight polymer.

A commercial example of the low molecular weight copolymer is Licocene^{®} PP 1602 from Clariant. Licocene PP 1602 is sold as granules and is described as a low melting, metallocene-technology based propylene-ethylene copolymer, which exhibits a low degree of crystallinity. The Mp of Licocene^{®} PP 1602 was measured to be 75,900 g/mol. Another example is Licocene^{®} PP 1302. The Mp of Licocene^{®} PP 1302 was measured to be 24,100 g/mol. Licocene^{®} PP 3602 which is sold as granules and is described as a low crystalline metallocene-catalyzed propylene-ethylene copolymer may also be used. Further examples of low molecular weight polymers are Vistamaxx 8780 and Vistamaxx 8880 from Exxon. A blend of two or more low molecular weight metallocene-catalyzed propylene-ethylene copolymers may be used, as blending two lower molecular weight copolymers with different crystallinity was found to enable low stiffness while still maintaining high toughness. An example is a blend of Licocene^{®} 3602 and Licocene^{®} 1602, which are both propylene-ethylene copolymers from Clariant. Licocene 3602 is a relatively highly crystalline polymer while Licocene 1602 has a medium crystallinity. In a blend of both (e.g. 2:1 for the ratio of Licocene 1602 to Licocene 3602) the overall crystallinity can be adjusted in a way that the resulting hotmelt composition provide good anchoring points of the cellulose fibers. Another example is a 2:1 blend of Licocene 1602 and Licocene 2602.

The Toughness of the formulation can be significantly increased when a polyolefin having a high peak molecular weight Mp of from 130,000 g/mol to 700,000 g/mol, is added in the formulation. The high molecular weight polyolefin may have a peak molecular weight which is at least greater by 10,000 g/mol than the peak molecular weight of the low molecular weight metallocene-catalyzed polyolefin(s) described above (the highest peak for blends), in particular at least 20,000 g/mol, or even at least 50,000 g/mol greater. The high molecular weight polyolefin may in particular have a peak molecular weight of from 140,000 g/mol to 410,000 g/mol, or from 150,000 g/mol to 360,000 g/mol.

The inventors have found that, surprisingly, the addition of a longer molecular weight polyolefin significantly increases the strain hardening of the blend besides increasing the elongation at break, which in combination results in a significantly higher Toughness of the formulation. Strain hardening is believed to be a "self-repairing mechanism of the blend when being strained, which avoids early rupture.

The higher molecular weight polymer, if present, may be advantageously comprised of a single material to simplify the compounding and formulation of the hotmelt material, but it may also be a blend of individual polymers. The hotmelt material may for example comprise from 1% to 20% of such a high molecular weight polymer(s), by weight of the hotmelt material. Already small additions of the longer molecular weight polymers may boost the strain hardening and hence the Toughness of the hotmelt material. More than 10% by weight may on the other hand increase the viscosity. The higher molecular weight polyolefins may be a homopolymer or a copolymer. The copolymer may comprise different alpha olefin monomers such as ethylene, propylene, 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof.

The higher molecular weight polymers may be in particular a propylene-ethylene copolymer. The high molecular weight polyolefin may also be a metallocene-catalyzed based copolymer, in particular a metallocene-catalyzed propylene-ethylene copolymer. The high molecular weight polyolefin may in particular be a propylene-ethylene copolymer comprising greater than 80 wt.% of polypropylene units with isotactic stereochemistry. Examples of such copolymers are commercially available as the Vistamaxx series from ExxonMobil. For example, Vistamaxx 6202 and Vistamaxx 6502 are sold as pellets and are described by their manufacturer as primarily composed of isotactic propylene repeat units with random ethylene distribution, produced using a metallocene catalyst technology. Vistamaxx 6202 and 6502 can be used as high molecular weight polymers in the formula examples below. Vistamaxx 6502 has the lowest viscosity, and thus the least impact on increasing the viscosity of the total composition.

Nonlimiting examples of commercially available higher molecular weight polymers are Affinity EG 8200G, Engage 8200, Infuse 9817, Vistamaxx 3000, Vistamaxx 6102, Vistamaxx 6202, Vistamaxx 6502, VERsify 4200, VERsify 4301. The higher molecular weight polymer may also be a block copolymer styrene-butadiene also known as styrene-butadiene copolymer (SBC). Kraton MD 1648 is a commercial example of SBC that may be used in the hotmelt material of the present invention.

A hotmelt composition according to the invention may also be advantageously formulated with a single polymer that enables the desired properties of the laminate while reducing complexity of the formulation. A metallocene-catalyzed propylene-ethylene copolymer under the tradename Licocene PP 2502 having a peak molecular weight of 57,100 g/mol may be used. Another commercial example of polymer that may be used as single polymer backbone for the hotmelt composition is Vistamaxx 8780, however it is less preferred due to the higher viscosity of Vistamaxx 8780.

Optional components may be further added to the hotmelt material, as is known in the art, in particular tackifiers, plasticizers, antioxidants, hydrophilic melt additives and the like.

Tackifiers otherwise called "tackifier resins" or "tackifying resins" are low-molecular weight compounds (oligomers) that are added to adhesive formulations to improve tack and peel adhesion materials. Usual tackifiers known in the art may be used in the present invention. Typical tackifiers are thermoplastic materials stable at least up to 200°C, being amorphous glasses at room temperature, and having a Tg higher than 50 °C, preferably comprised between 80 °C and 125 °C. Tackifiers typically have a molecular weight comprised between 500 and 2000 Daltons.

Tackifiers are in general organic chemicals with polycyclic structure. Commonly used tackifiers are selected from rosin resins and their derivatives (rosin esters), hydrocarbon resins produced from petroleum-based by-products of naphtha crackers, and terpene resins (modified or not). Hydrocarbon resins may be aliphatic, cycloaliphatic and aromatic resins (in particular C5 aliphatic resins, C9 aromatic resins, and C5/C9 aliphatic/aromatic resins), and may be optionally hydrogenated hydrocarbon resins.

Exemplary tackifiers include aliphatic hydrocarbon resins, aromatic modified aliphatic hydrocarbon resins, hydrogenated poly-cyclopentadiene resins, poly-cyclopentadiene resins, gum rosins, gum rosin esters, wood rosins, wood rosin esters, tall oil rosins, tall oil rosin esters, poly-terpenes, aromatic modified poly-terpenes, terpene-phenolics, aromatic modified hydrogenated poly-cyclopentadiene resins, hydrogenated aliphatic resins, hydrogenated aliphatic aromatic resins, hydrogenated terpenes and modified terpenes, and hydrogenated rosin esters. Particularly suitable tackifiers are rosin (and its derivatives) resins and hydrogenated hydrocarbon tackifiers, which are solid at room temperature.

However, the hotmelt material of the invention has the advantage that it may be relatively simply formulated with low amount of tackifiers (below 40% by weight, in particular below 20% or even below 10% by weight), or even be free of tackifiers. There are several advantages to hotmelt material with low amount or no tackifier. Firstly, as indicated, this reduce the complexity of the formulation and for example facilitate quality control and traceability of the ingredients. Reducing or eliminating tackifiers in the hotmelt material and the hotmelt coating composition further allows forming a roll 36 of the laminate 40 without the risk of the laminate sticking to itself.

The hotmelt material may optionally comprise an antioxidant. Non-limiting examples of suitable antioxidants include amine-based antioxidants such as alkyl diphenyl amines, phenyl-naphthylamine, alkyl or aralkyl substituted phenyl-naphthylamine, alkylated p-phenylene diamines, tetramethyl-diaminodiphenylamine and the like; and hindered phenol compounds such as 2,6-di-t-butyl-4-methylphenol; 1,3,5-trimethyl-2,4,6-tris(3',5'-di-t-butyl-4'-hydroxybenzyl)benzene; tetra kis [(methylene(3,5-di-t-butyl-4-hydroxyhydrocinnamate)]methane (e.g., IRGANOXTM 1010, from Ciba Geigy, New York); octadecyl-3,5-di-t-butyl-4-hydroxycinnamate (e.g., IRGANOXTM 1076, commercially available from Ciba Geigy) and combinations thereof. When used, the amount of the antioxidant in the hotmelt material can be respectively less than 1 %, alternatively from about 0.05 % to about 0.75 %, and alternatively from about 0.1 % to about 0.5 %, by weight of the hotmelt adhesive.

The hotmelt material may optionally comprise a UV stabilizer that may prevent or reduce the degradation of the composition by radiation. Any UV stabilizer known to a person of ordinary skill in the art may be used in the hotmelt composition. Non-limiting examples of suitable UV stabilizers include benzophenones, benzotriazoles, aryl esters, oxanilides, acrylic esters, formamidine carbon black, hindered amines, nickel quenchers, hindered amines, phenolic antioxidants, metallic salts, zinc compounds, and combinations thereof. Where used, the amount of the UV stabilizer in the hotmelt material can be less than 1 %, alternatively from about 0.05 % to about 0.75 %, and alternatively from about 0.1 % to about 0.5 %, by weight of the hotmelt material.

The hotmelt material may optionally comprise a fragrance such as a perfume or other odorant. Such fragrances may be retained by a liner or contained in release agents such as microcapsules that may, for example, release fragrance upon removal of a release liner from or compression on the adhesive composition. Where used, the amount of the fragrance in the hotmelt composition can be less than 3 %, alternatively less than 2 %, alternatively less than 1 %, alternatively from about 0.05 % to about 0.75 %, and alternatively from about 0.1 % to about 0.5 %, by weight of the hotmelt composition.

The hotmelt material may optionally comprise a hydrophilic additive. As for the other components indicated, the hotmelt material may comprise one, two or more, of such hydrophilic additives. Hydrophilic melt additives are amphiphilic molecule having a hydrophilic head and a hydrophobic tail. The hydrophilic head is oriented towards the surface of the adhesive, thus providing for the hydrophilic character of the hotmelt material, while the hydrophobic head remains in the polymer matrix. The hotmelt material may have particularly stable hydrophilic properties, enabled by the compatibility of the metallocene-catalyzed polyolefin and the hydrophilic melt additive.

Hydrophilic melt additives are typically compounded in a masterbatch in the form of pellets than can be incorporated by homogenous mixing in the molten polyolefin. Commercial examples of hotmelt additives particularly compatible with a propylene-based metallocene-catalyzed polyolefin are PPM 15560 from Techmer (hydrophilic PP masterbatch) and Brij S2 (Croda). Further, in order of declining preference, Brij S10 (from Croda,) Unithox 450, Unithox 720 and Unithox 750 (from Baker Hughes) can be used. PPM 15560 is preferably used in a dosage of 0.5 weight percent of the masterbatch, Brij S2 and Brij S 10 in a dosage of preferably 2 weight percent of the active. Techsurf^{®} melt additives from Techmer have been used to impart hydrophilicity to polyolefin fibers, nonwoven fabrics, and specialty plastic applications, and are useful in the present invention.

The additives of the Brij^{®} series from Croda are ethoxylated alcohols of the general formula: 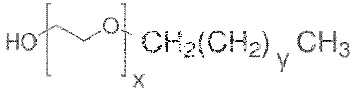 with x ranging from 2 to 100 and y ranging from 12 to 24, in particular y = 16 (stearyl).

For example, Brij S2 with x = 2 and y = 16 has a low molecular weight of 386 g/mol, which presumably facilitates the diffusion to the surface. These ethoxylated alcohols may be a more cost-effective alternative to the above mentioned blend. The blends described in US6,146,757 were found to enable a stronger hydrophilic effect, while Brij S2 enables a milder hydrophilic effect. One or the other additive may be thus preferred depending on the application purpose. In some applications (such as bonding of the topsheet to the acquisition layer), a milder hydrophilicity may be preferred to facilitate the draining of the top layer by lower layers in the diaper, and avoid liquid remaining in the top layer being exposed to the consumer.

A low contact angle is desirable so that water, urine or other water-based discharges "wet out" rather than "bead up" resulting in the fluid being directed into the absorbent core and away from the topsheet. The hydrophilicity of a hotmelt material can be quantified by the Contact Angle Method described hereinafter. The hotmelt materials can thus, if desired, exhibit a Fresh Film Contact Angle and an Aged Film Contact Angle with distilled water of less than 70 degrees, preferably of less than 50 degrees, as measured by the Contact Angle Test described herein.

The hotmelt material may also advantageously have a storage modulus (G') that is higher than 0.3 MPa at 37°C, as measured with Oscillatory Rheometry Test Method described below. The relatively high G' value is indicative of a polymeric composition that is not tacky in this range of temperature. A non-tacky behavior enables direct winding as a roll of good material when the external surface of the laminate is coated with a hotmelt coating composition 32. It also contributes to avoid process contaminations.

The hotmelt material may typically have a storage modulus (G') higher than 10 MPa at 37°C, or even 15 MPa at 37°C. The storage modulus (G') may on the other be typically lower than 200 MPa at 37°C. The hotmelt material has preferably a Storage Modulus (G') at 37°C between 3 MPa and 40 MPa.

The hotmelt material may also have a storage modulus higher than 0.3 MPa at 23°C, or even higher than 15 MPa at 23°C, or even higher than 20 MPa at 23°C. On the other hand, the hotmelt material may also have a storage modulus that is lower than 300 MPa at 23°C. Typically the G' value of polymers decreases when the temperature increases. The hot-melt adhesive may also have a storage modulus (G') higher than 0.3 MPa at 60°C.

Commercially available propylene-ethylene copolymers such as those from Clariant's Licocene have the following G', as measured as indicated below:

| Hot Melt Material | Storage modulus (G') at 37°C (MPa) | Storage modulus (G') at 23°C (MPa) |
|---|---|---|
| Licocene PP 1502 | 10 | 14 |
| Licocene PP 1602 | 8.6 | 12 |
| Licocene PP 2502 | 33 | 52 |
| L-MODU S 410 (PP homopolymer ex. Idemitsu) | 9.1 | 14 |
| Blend of 90% Licocene 2502 and 10% Vistamaxx 6502 | 26 | - |
| Blend of 95% Licocene 2502 and 5% Vistamaxx 6502 | 29 | - |
| Blend of 90% Licocene 2502 and 10% of the hydrogenated tackifier Eastotac Resh H-100L | 20 | - |

The table provides viscosities at 170°C for one-polymer component hotmelt materials suitable for the present invention:

| Hotmelt Material | Viscosity [mPas] at 170°C |
|---|---|
| Licocene PP 1602 | 5,900 |
| Licocene PP 2502 | 1,900 |
| Licocene PP 2602 | 6,800 |

The following table provides viscosities at 150°C of further hotmelt materials suitable for the present invention, and estimated values at 170°C:

| Hot Melt Material | Viscosity [mPas] at 150°C | Viscosity [mPas] at 170°C (estimated) |
|---|---|---|
| 66.6% Licocene 1602 / 33.3% Licocene 2602 | 9,000 | 4,500 |
| 90% Licocene 2502 / 10 % Vistamaxx 6502 | 9,670 | 4,800 |
| 95% Licocene 2502 / 5 % Vistamaxx 6502 | 6,000 | 3,000 |
| 90% Licocene 2502 / 10 % Tackifier Eastotac Resh H-100L | 3,360 | 1,680 |

### Absorbent article 41

"Absorbent articles", as used herein, refers to personal hygiene products that are placed against or in proximity to the body of the wearer to absorb and contain the various exudates discharged from the body. Absorbent articles include baby diapers, training pants, adult incontinence undergarments, feminine hygiene products, disposable inserts for hybrid diapers, bed matt, changing matt and the like. As used herein, the term "body fluids" or "body exudates" includes, but is not limited to, urine, blood, vaginal discharges and fecal matter. These articles are typically disposable, meaning are not intended to be laundered or otherwise restored or reused as a hygienic article after a single use. Disposable absorbent articles typically comprise a liquid pervious topsheet, a liquid impervious backsheet and an absorbent core positioned between the topsheet and the backsheet. The laminate of the present invention may be used in such an absorbent article as an acquisition-distribution system between the topsheet and the absorbent core.

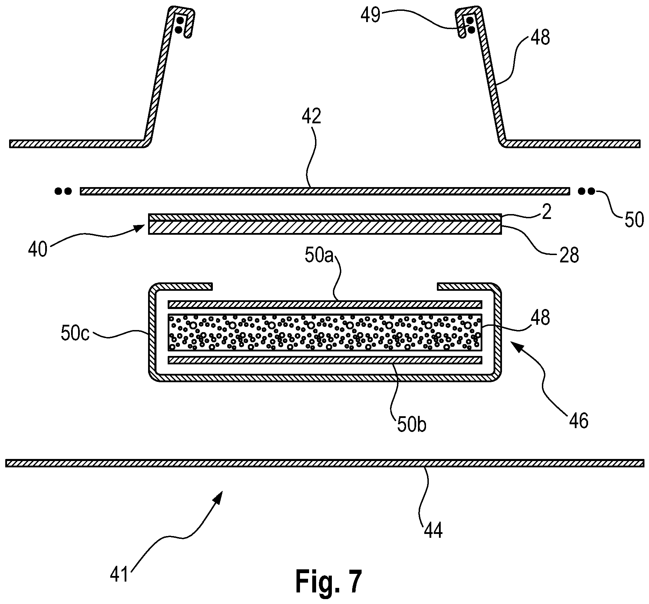

Fig. 7 shows a schematic cross-section of a diaper 41 as exemplary absorbent article comprising a topsheet 42, backsheet 44 and absorbent core 46, which are discussed in more details below. The absorbent article 41 further comprises the laminate 40 of the invention as an absorption-distribution system disposed between the topsheet and the absorbent core. In this application, the substrate 2 is preferably a liquid permeable nonwoven, which may have been further treated by a hydrophilic agent to facilitate the acquisition of urine or other fluid from the topsheet. The fibrous layer 28 takes the role of a distribution layer, as the capillarity provided by the pores between the cellulose fibers can quickly acquire and distribute the fluid, passing it to the absorbent core. The hotmelt material 14 by bonding and locking the cellulose fibers in the first thickness of the fibrous layer provides integrity to the fibrous layer on the internal side of the fibrous layer. This enables the acquisition-distribution system of the invention to keep its functionality, avoiding the fibrous structure to lose it structure. Meltblown/spunmelts fibers 16 if present also contribute to the stability of the laminate.

In order to provide favorable fluid handling performance, the substrate layer 2 may have higher permeability (as measured according to industry standard test WSP 70.1 (20)) and lower liquid wicking rate (according to industry standard test WSP 10.1 (20)) when compared to the permeability and liquid wicking rate of the laminate as a whole.

A short description of the other components of a typical absorbent articles, in particular a taped or pant diapers now follows. The topsheet 42 is the part of the absorbent article 41 that is in contact with the wearer's skin. The topsheet may be joined to portions of the backsheet 44, the absorbent core 46, the barrier leg cuffs 48, and/or any other layers as is known to those of ordinary skill in the art. The topsheet may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured, may have any suitable three-dimensional features, and/or may have a plurality of embossments (e.g., a bond pattern). The topsheet may be apertured by overbonding a material and then rupturing the overbonds through ring rolling, such as disclosed in US5,628,097 (Benson et al.), and disclosed in US2016/0136014 (Arora et al.). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The backsheet 44 is generally that portion of the absorbent article 41 positioned proximate to the garment-facing surface of the absorbent core 46. The backsheet may be joined to portions of the topsheet, the absorbent core 30, and/or any other layers of the absorbent article by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

The outer cover material (sometimes referred to as a backsheet nonwoven) may comprise one or more nonwoven materials joined to the backsheet 44 and that covers the backsheet 44. The outer cover material forms at least a portion of the garment-facing surface of the absorbent article and effectively "covers" the backsheet so that the film is not present on the garment-facing surface. The outer cover material may comprise a bond pattern, apertures, and/or three-dimensional features. The outer cover material may be a hydroentangled nonwoven material.

As used herein, the term "absorbent core" 46 refers to a component of the absorbent article 41 disposed in the article for absorbing and containing liquid such as urine received by the absorbent article. The absorbent core thus typically has a high absorbent capacity. The absorbent core comprises an absorbent material 48, that is typically enclosed within or sandwiched between a core wrap 50a, 50b.

The core wrap may be a single material that is folded and attached to itself, or it may comprise a separate top layer 50a and bottom layer 50b that may be bonded together. An overwrap 50c may also be present, especially for absorbent core comprising an absorbent layer substantially free of cellulose fibers. The absorbent material 48 typically comprises superabsorbent particles which are optionally mixed with cellulose fibers. As used herein, "absorbent core" does not include any acquisition-distribution systems, topsheet, or backsheet of the absorbent article.

The absorbent material 48 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists of SAP. The absorbent material may also be a high internal phase emulsion (HIPE) absorbent foam.

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from 20 g/g to 40 g/g. "Superabsorbent polymer particles", as used herein, refers to a superabsorbent polymer material which is in particulate form so as to be flowable in the dry state.

Various absorbent core designs comprising high amount of SAP have been proposed in the past, see for example in US5,599,335 (Goldman), EP1,447,066 (Busam), WO95/11652 (Tanzer), US2008/0312622A1 (Hundorf), WO2012/052172 (Van Malderen). In particular the SAP printing technology as disclosed in US2006/024433 (Blessing), US2008/0312617 and US2010/0051166A1 (both to Hundorf et al.) may be used. The invention is however not limited to a particular type of absorbent core. The absorbent core may also comprise one or more glue such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate such as a carded nonwoven layer, having a porous structure into which SAP particles have been deposited, may also be used in present invention.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally extending channels, which are areas of the absorbent layer substantially free of absorbent material within the absorbent material layer. The top side of the core wrap may be advantageously bonded to the bottom side of the core by adhesive, mechanical or ultra-sonic bonding through these material-free areas. Exemplary disclosures of such channels in an airfelt-free core can be found in WO2012/170778 (Rosati et al.) and US2012/0312491 (Jackels). Channels may of course also be formed in absorbent cores comprising a mix of cellulose fibers and SAP particles. These channels can embody any suitable shapes and any suitable number of channels may be provided. In other instances, the absorbent core may be embossed to create the impression of channels. Absorbent cores with or without channels are also within the scope of the present disclosure.

The absorbent article 48 may comprise one or more pairs of barrier leg cuffs 48 and one or more pairs of leg elastics 50. The barrier leg cuffs 48 may be positioned laterally inboard of leg elastics 50. Each barrier leg cuff 48 may be formed by a piece of material which is bonded to the absorbent article 41 so it can extend upwards from a wearer-facing surface of the absorbent article and provide improved containment of body exudates approximately at the junction of the torso and legs of the wearer. The barrier leg cuffs 48 are delimited by a proximal edge joined directly or indirectly to the topsheet and/or the backsheet and a free terminal edge, which is intended to contact and form a seal with the wearer's skin. The barrier leg cuffs may extend at least partially between the front end edge and the back end edge of the absorbent article on opposite sides of the central longitudinal axis and may be at least present in the crotch region. The barrier leg cuffs 48 may each comprise one or more elastics 49 (e.g., elastic strands or strips) near or at the free terminal edge. These elastics 49 cause the barrier leg cuffs 48 to form a seal around the legs and torso of a wearer. The leg elastics 50 extend at least partially between the front end edge and the back end edge. The leg elastics 50 essentially cause portions of the absorbent article proximate to the chassis side edges to help form a seal around the legs of the wearer. The leg elastics may extend at least within the crotch region.

The absorbent articles may further comprise further common features for the type of absorbent articles, such as but not limited to, front and/or transversal barrier cuffs or waistbands, urine indicator that changes color when contacted with urine, fastening system with taped back ears and frontal landing zone, etc..

### Examples and data

A laminate according to the invention was formed by the process generally shown in Fig. 1 using the following starting material.

| | Substrate layer (2) | Glue (6) | Hotmelt (14) or Meltblown | Pulp (12) | Spunmelt (16) or Meltblown | Coating composition (32) | Total Basis Weight | Caliper (0.5 kPa) |
|---|---|---|---|---|---|---|---|---|
| Laminate example | 30 gsm Z87G ATB | 2 gsm Henkel581 3U | 4 gsm Licocene 2502 | 45 gsm | 6 gsm PP meltblown | 3 gsm Licocene 2502 | 90 gsm | 1.11 mm |
| Comparative example | 30 gsm Z87G ATB | 2 gsm Henkel581 3U | 4 gsm PP meltblown | 45 gsm | 6 gsm PP meltblown | 3 gsm Licocene 2502 | 90 gsm | 1.11 mm |

Z87G is an air-through bonded acquisition layer material available from Yanjan nonwovens. It comprises 60% PE/PET (2 dpf, denier per filament), and 40% PE/PET fibers (4 dpf). The hotmelt material was pure Licocene 2502 from Clariant, with 0.02% (by weight) Hostavin N30 added as antioxidant. The meltblown PP material comprise 3% by weight of Croda Brij S2 hydrophilic melt additive.

The comparative laminate's only difference is that the hotmelt material (Licocene 2502) was replaced by the same polypropylene material (at 4 gsm) used for layer 16. Thus, in the comparative laminate, meltblown fibers were sprayed within the stream of cellulose fibers from both sides, as in a conventional spinform process.

The drapability of the example laminate and the comparative laminate, as well as the air permeability were measured. The drapability was measured by letting a piece of 100 mm laminate hang from a support and measuring the vertical drop (according to the Horizontal Bending Drop test disclosed in WO2020/205485).

| | Drapability (mm) | Air Permeability (m³/m²/min) |
|---|---|---|
| Laminate example | 69 | 44.7 |
| Comparative example | 63 | 31.5 |

The inventive example had higher Drapability and Air Permeability values than the comparative example.

Both laminates (inventive and comparative) were used as acquisition-distribution system in Size 4 Hadaeno Ichiban premium taped diapers (marketed in China by P&G), replacing the commercial acquisition-distribution system comprising the cross-linked cellulose fiber patch discussed in the "Background Section". The laminates were placed such that substrate layer was oriented towards the topsheet of the diaper, and the fibrous layer towards the absorbent core. The topsheet was a 22 gsm apertured carded topsheet, and the absorbent core comprised 12.5 g of SAP and was free of cellulose fibers.

The acquisition time for 4 succeeding gushes of 75ml saline water (0.3 psi loading) and the resulting Rewet were measured in lab condition according to a standard protocol (C-GAM, Curved Global Acquisition Method) for the inventive diaper, the comparative diaper and the commercial diaper indicated above. The results were as follow:

| | | 1st Gush acquisition time (sec) | 2nd Gush acquisition time (sec) | 3rd Gush acquisition time (sec) | 4th Gush acquisition time (sec) | Rewet (9) |
|---|---|---|---|---|---|---|
| Inventive Diaper | Average | 69.2 | 68.1 | 94.2 | 272.0 | 0.176 |
| | Standard Deviation | 4.1 | 8.7 | 13.1 | 96.0 | 0.012 |
| Comparative Diaper | Average | 67.6 | 80.6 | 133.1 | 437.6 | 0.165 |
| | Standard Deviation | 2.8 | 8.3 | 16.4 | 121.3 | 0.01 |
| Commercial Diaper | Average | 58.5 | 79.0 | 127.9 | 324.3 | 0.138 |
| | Standard Deviation | 1.8 | 6.0 | 10.5 | 64.5 | 0.011 |

The inventive laminate resulted in improved fluid handling properties, while maintaining the dryness vs. the comparative laminate.

### Dust Data:

In order to assess the tendency of a material to generate dust, a pre-defined amount of material (90 m²) is unwound and run over an idler with a 90° wrap angle at a given speed of 100 m/min. The dust generated due to friction between the material and idler is collected and weighed for comparison. The assessment was conducted for one the laminate of the inventive example above and the comparative example indicated above.

| | Dust [g] | Dust Reduction [%] |
|---|---|---|
| Inventive example | 0.0349 | 45 |
| Comparative example | 0.0633 | - |

This Example demonstrates that using an hotmelt according to the invention provides the additional benefit of reduced dust compared to a spinform web.

### Other examples

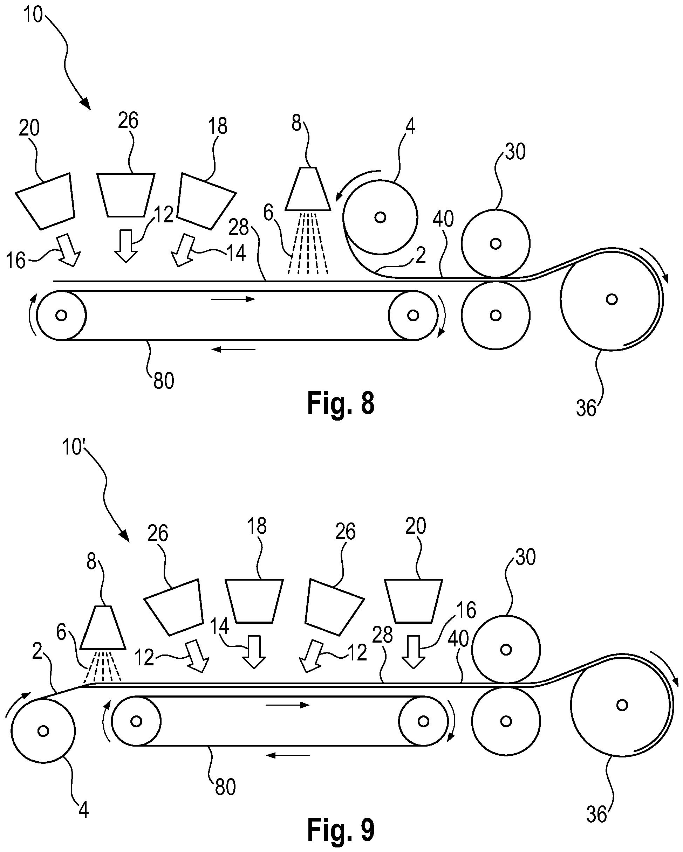

While the laminate of the invention and a process to make it have been described with reference to the exemplary embodiments of Figs. 1 - 6, these are not exhaustive. Further non-limiting embodiments of the present invention are described herein below with reference to the schematic Figures 8-10.

Fig. 8 shows a variation of the process disclosed in Fig. 1, wherein the fibrous layer 28 is first deposited on an endless rotating belt 80 before being bonded to the substrate layer 2 by a layer of adhesive 6. The continuous belt may be made of PET, nylon or any other material fit for the purpose. A drum with a foraminous surface connected to a negative pressure source may also be used as is known in the art.

Fig. 9 shows another variation of the process disclosed in Fig. 1, wherein instead of using a conventional spinform deposition device 10, an alternative deposition device 10' is used wherein the hotmelt material 14 is sprayed from a nozzle 18 disposed between two spout 26 forming each a stream of cellulose fibers 12. The hotmelt material and the cellulose fibers intermix before being deposited as a fibrous layer, for example on a substrate 2 which has been preferably be coated with a layer of adhesive 6. An optional spunmelt layer 16 may be sprayed on the deposited fibrous layer 28. The hotmelt lumps 17 may be formed through the whole thickness of the fibrous layer 28 or in a central section of the fibrous layer, depending on operating conditions such as the incident angles of the streams of cellulose fibers, the speed of the carrying medium, the amount of material sprayed etc..

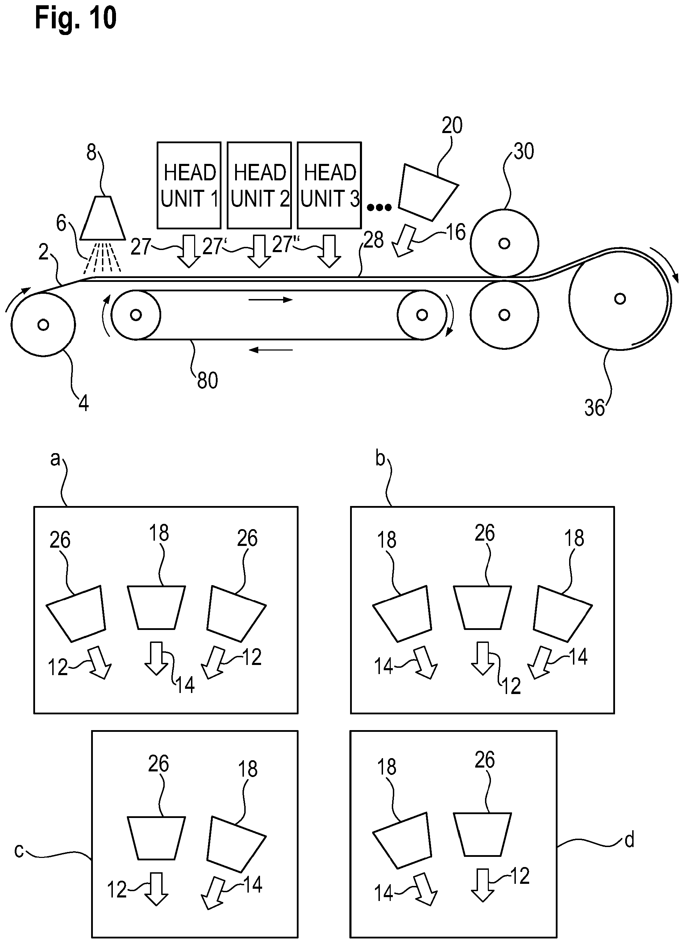

Fig. 10 shows another variation of the process, wherein two or more deposition devices ("head units" 1, 2, 3..) are disposed in series and each subsequently deposit a stream of fibrous layer 27, 27', 27", typically on the substrate 2 or the belt 80 as described previously. This process with multiple heads allows the formation of more complex laminate structure. Each deposition **device** may be for example as schematically exemplified by the head units a..d shown at the bottom of Fig. 10. Head unit "a" as shown may be a deposition device 10' as in Fig. 9, wherein the hotmelt material 14 is sprayed between two stream of cellulose fibers. Head unit "b" shows a fibrous deposition device 10 as shown in Fig. 1. Head units "c" and "d" only comprise a cellulose fibers spout 26 and a hotmelt material nozzle 18. These head units a-d may also be used alone as deposition devices to make a laminate according to the invention. An optional meltblown layer or spunbond layer 16 may be deposited on the fibrous layer 28 as described before in Fig. 9 or mounted on the last head unit. A calendar roll 30 may be used in all these additional examples to further stabilize the laminate 20. A coating device (not represented) may be optionally present after the calendaring step, as described in relation with Fig. 1 to reduce fibrous dusting.

### Test Methods

### Viscosity Test Method

The Viscosity of a hot melt adhesive composition is determined by performing ASTM D3236-15 with the following additional guidance. A Brookfield RVT viscometer with spindle SC 4-27 (Brookfield Engineering, Middleboro, MA, USA), or equivalent, is used. The sample temperature is maintained at the desired temperature (170.0 ± 1.0 °C) throughout the measurement. The sample is preheated for 10 minutes and stirred with the measurement spindle for 30 min. The spindle is rotated at 20 rpm throughout the measurement. The resulting apparent viscosity, as described in section 10, is reported as the "viscosity" in units of millipascal-seconds to the nearest 100 mPa·s.

### Peak Molecular Weight (Mp) Measurement Method

The peak molecular weight is determined using a gel permeation chromatography (GPC) method. GPC is a well-known method wherein polymers are separated according to molecular size, the largest molecule eluting first. The peak molecular weights referred to herein can be determined with gel permeation chromatography (GPC) using polystyrene calibration standards, such as is done according to ASTM D5296. The molecular weight of any polymer or unknown polymer measured using GPC so calibrated is the styrene equivalent molecular weight, which herein is defined as the "peak molecular weight." Suitable solvents and temperatures are employed with GPC in order to achieve adequate molecular weight separation and resolution.

### Oscillatory Rheometry Test Method

The Oscillatory Rheometry Test Method is used to measure the Storage Modulus and the Loss Factor of a hotmelt composition. A controlled-stress rotational rheometer (such as Discovery **HR-3, TA** Instruments, New Castle, DE, USA, or equivalent) capable of sample temperature control (using a Peltier cooler and resistance heater combination) with a precision equal to or exceeding 0.5 °C over at least the range of -10 °C to 150 °C. The rheometer is operated in a parallel plate configuration with 20-mm stainless steel parallel-plate tooling.

A parallel plate gap of 1000 µm is initially used in the method. To compensate for thermal expansion of the tooling, the gap is set to 1000 µm, and a mapping of actual plate gap (as measured using a suitable standard test fluid) a function of temperature over the range -10 °C to 150 °C is performed. This mapping is then used throughout the determination of the Storage Modulus Parameter and the Loss Factor Parameter.

The rheometer is heated to 150 °C, hot melt adhesive composition is introduced in the rheometer, the gap is set to 1050 µm, excess protruding sample is trimmed, and the gap is then set to 1000 µm. (The axial force control of the rheometer is set to be maintained within ± 0.1 N of force, and the thermal expansion/contraction of the sample itself is compensated in order to avoid overfilling or underfilling of the gap in addition to the abovementioned compensation of the tooling.) The rheometer is then allowed to cool to 130 °C, at which point the measurement commences with temperature ramped from 130 °C to -10 °C at a constant rate of cooling of 2 °C/min. The applied strain amplitude is 0.1%, and the frequency of oscillation is 1 Hz (that is, one cycle per second). The resulting oscillatory stress is recorded.

After this step, the sample temperature is set to 23 °C (temperature is ramped to this setpoint at a rate of 10 °C/min), and the sample is allowed to rest for 4.0 hours at 23°C. At the end of this period, the temperature is set to -10 °C (temperature is ramped to this setpoint at a rate of 10 °C/min), the sample is equilibrated for 300 seconds at -10 °C, and a second oscillatory rheology measurement is conducted (0.1% strain, frequency of oscillation of 1 Hz) while temperature is ramped upward to 130 °C at a constant rate of increase of 2 °C/min.

From the second, increasing temperature sweep, the storage modulus G' is calculated and recorded at 23 °C and 37 °C, and these values are reported in megapascals (MPa) to the nearest 0.01 MPa as the "Storage Modulus at 23 °C" and the "Storage Modulus at 37 °C," respectively. From the second, increasing temperature sweep, the loss factor (also known as tan delta) is calculated recorded at 23 °C and 37 °C, and these dimensionless values are reported to the nearest hundredth as the "Loss Factor at 23 °C" and the "Loss Factor at 37 °C," respectively.

### Thickness Measurement Method

This method is used to measure the thickness (caliper) of a nonwoven or laminate according to the invention.

Equipment: Mitutoyo manual caliper gauge with a resolution of 0.01 mm, or equivalent instrument.

Contact Foot: Flat circular foot with a diameter of 56.0 mm (± 0.2 mm). A circular weight may be applied to the foot (e.g., a weight with a slot to facilitate application around the instrument shaft) to achieve the target weight. The total weight of foot and added weight (including shaft) is selected to provide 0.5 kPa of pressure to the sample.

The caliper gauge is mounted with the lower surface of the contact foot in a horizontal plane so that the lower surface of the contact foot contacts the center of the flat horizontal upper surface of a base plate approximately 20 x 25 cm. The gauge is set to read zero with the contact foot resting on the base plate.

Ruler: Calibrated metal ruler graduated in mm.

Stopwatch: Accuracy 1 second.

Sample preparation: The layer to be measured is conditioned at least 24 hours as indicated above.

Measurement procedure: The layer is laid flat. For a laminate, the substrate layer is placed facing down. The point of measurement, i.e. the middle of the sample, is carefully drawn on the top side of the layer, taking care not to compress or deform the layer.

The contact foot of the caliper gauge is raised and the layer is placed flat on the base plate of the caliper gauge with the top side up so that when lowered, the center of the foot is on the marked measuring point.

The foot is gently lowered onto the sample and released (ensure calibration to "0" prior to the start of the measurement). The caliper value is read to the nearest 0.01 mm, 10 seconds after the foot is released.

The procedure is repeated for at least 5 samples measured in this manner for a given material and the average thickness is calculated and reported with an accuracy of one tenth mm.

### Contact Angle Method

The Contact Angle Method is used to measure the contact angle of deionized water on a thin film of hotmelt adhesive after 24 hours ("Fresh Film Contact Angle) and accelerated ageing ("Aged Film Contact Angle). Contact angles of deionized water (resistivity 18.2 MΩ or greater at 25 °C) on sample adhesive substrate are determined using the sessile-drop approach generally described in ASTM D7490-13 using a goniometer and appropriate image acquisition system. The goniometer stage is leveled, and the image acquisition system is configured so as to capture toward the center of the stage, along an axis that is in the plane of the stage. The image acquisition system is further configured such that a droplet delivered to the center of the stage is in focus. Contact-angle analysis is carried out under a laboratory environment of 23 ± 2 °C and 40 ± 10% relative humidity.

A continuous adhesive film (at least 50 micrometer thick) is coated on a smooth substrate (e.g. silicone-coated paper) by a hotmelt coater at 170°C (adapt temperature if needed). The film is then cooled down to ambient lab conditions (21-23°C, 40%-60% RH). The contact angle testing is conducted on the adhesive surface 24 hour after the adhesive coating making.

Five specimen locations from one or more like regions of a sample adhesive are analyzed. Sufficiently many like sample adhesive regions required to provide five specimens 1 cm × 1 cm in dimension are selected. The five specimens are excised from the sample adhesive regions. Each specimen is analyzed by first placing it on the leveled goniometer stage. A flat-tipped, 14-gauge needle (outer diameter 2.11 mm) is then used to deliver a 25.0 ± 0.05 µL droplet of deionized water on the center of the specimen. An image of the droplet is captured 5.0 seconds after delivery. Tangent lines are drawn at both horizontal extrema of the imaged droplet, and the angle containing the droplet-substrate interface between each tangent line and the horizontal substrate is measured. The arithmetic mean of these two angles is the contact angle for that specimen and is recorded to the nearest 0.1°. The contact angles for the five specimens are determined in this way. The arithmetic mean of the five specimen contact angles is calculated and recorded as the Fresh Film Contact Angle.

The Aged Film Contact Angle is obtained in similar manner with the difference is that the adhesive film is aged at ambient pressure and at 60 ± 3 °C for 16 days. Subsequently, the adhesive film is kept at 23 ± 2 °C and 40 ± 10% relative humidity for 24 hours immediately prior to contact-angle analysis as indicated above.

## Claims

1. A laminate (40) comprising a substrate layer (2) and a fibrous layer (28), each layer having an internal side and an external side, wherein the internal side of one layer faces the internal side of the other layer,
the fibrous layer comprising cellulose fibers (12) and irregularly shaped lumps (17) of hotmelt material (14), wherein these lumps of hotmelt material at least partially bond the cellulose fibers together, thus providing integrity to the fibrous layer.

2. A laminate (40) according to claim 1, wherein the lumps (17) of hotmelt material at least partially bond the cellulose fibers at least through a first thickness (13) of the fibrous layer adjacent the internal side of the fibrous layer.

3. The laminate according to any of the preceding claims, wherein the hotmelt material has a viscosity at 170°C ranging from 100 mPa.s to 10,000 mPa.s, as measured by the Viscosity Test Method disclosed herein.

4. The laminate according to any of the preceding claims, wherein the hotmelt material has a Storage Modulus (G') at 37 °C of from about 3 MPa to 40 MPa, as measured by the Oscillatory Rheometry Test Method disclosed herein.

5. The laminate according to any of the preceding claims, wherein the hotmelt material comprises a metallocene-catalyzed polymer having a peak molecular weight (Mp) in the range of from 10,000 g/mol to 130,000 g/mol, wherein the peak molecular weight is measured according to the Peak Molecular Weight Measurement Method disclosed herein, preferably wherein the hotmelt material comprises at least 50% by weight of the metallocene-catalyzed polymer.

6. The laminate according to any of the preceding claims, wherein the internal side of the substrate layer and the internal side of the fibrous layer are bonded by an adhesive (6), in particular an adhesive comprising a tackifier.

7. The laminate according to any of the preceding claims, wherein the fibrous layer (28) further comprises spunmelt fibers (16) intermixed with the cellulose fibers.

8. The laminate according to the preceding claim, wherein the spunmelt fibers are meltblown fibers (16), in particular polypropylene meltblown fibers.

9. The laminate according to any of the preceding claims, wherein the substrate layer (2) is a nonwoven layer, preferably wherein the nonwoven layer comprises a hydrophilic agent.

10. The laminate according to any of the preceding claims, wherein the laminate further comprises a fiber coating composition (32) that at least partially coats and binds the fibers on the external side of the fibrous layer.

11. A method for making a laminate according to any of the preceding claims, the method comprising the steps of:
- providing a substrate layer (2) having an internal side and an external side;
- optionally applying an adhesive (6) on the internal side of the substrate layer;
- providing a stream of cellulose fibers (12);
- spraying the hotmelt material (14) into the stream of cellulose fibers;
- optionally spunmelting fibers (16) into the stream of cellulose fibers; and then
- forming the fibrous layer (28) by depositing the combined stream (27) of cellulose fibers, hotmelt material and, if present, spunmelt fibers on the internal side of the substrate layer.

12. A method for making a laminate according to the preceding claim, the method further comprising the steps of:
- spraying a fiber coating composition (32) on the external side of the deposited fibrous layer so that the fiber coating composition at least partially coats and binds the fibers on this external side of the fibrous layer.

13. A method according to the preceding claim, wherein the fiber coating composition is a hotmelt material polymeric material, which may be the same of different as the hotmelt material as indicated in any of the preceding claims.

14. A personal hygiene absorbent article comprising a topsheet (42) on its wearer-facing side, an acquisition-distribution system (40), an absorbent core (46) and a backsheet (44) on its garment-facing side, wherein the acquisition-distribution system is a laminate according to any of the claims 1-10, wherein the substrate layer (2) is oriented towards the topsheet and the fibrous layer is oriented towards the absorbent core.

15. A personal hygiene absorbent article according to the preceding claim, wherein the absorbent core comprises a top layer (50a), a bottom layer (50b) and an absorbent layer (48) between the top layer and the bottom layer, wherein the absorbent layer comprises from 0% to 20% of cellulose fibers by weight of the absorbent layer.

## Patentansprüche

1. Laminat (40), umfassend eine Substratschicht (2) und eine Faserschicht (28), wobei jede Schicht eine Innenseite und eine Außenseite aufweist, wobei die Innenseite einer Schicht der Innenseite der anderen Schicht zugewandt ist,
die Faserschicht umfassend Cellulosefasern (12) und unregelmäßig geformte Klumpen (17) aus Hotmelt-Material (14), wobei diese Klumpen aus Hotmelt-Material die Cellulosefasern wenigstens teilweise miteinander binden, wobei somit der Faserschicht Integrität bereitgestellt wird.

2. Laminat (40) nach Anspruch 1, wobei die Klumpen (17) aus Hotmelt-Material die Cellulosefasern wenigstens teilweise wenigstens über eine erste Dicke (13) der Faserschicht binden, die benachbart zu der Innenseite der Faserschicht ist.

3. Laminat nach einem der vorstehenden Ansprüche, wobei das Hotmelt-Material eine Viskosität bei 170 °C aufweist, die von 100 mPa.s bis 10.000 mPa.s reicht, gemessen durch das hierin offenbarte Viskositätsprüfverfahren.

4. Laminat nach einem der vorstehenden Ansprüche, wobei das Hotmelt-Material einen Speichermodul (G') bei 37 °C von etwa 3 MPa bis 40 MPa aufweist, gemessen durch das hierin offenbarte Oszillationsrheometrieprüfverfahren.

5. Laminat nach einem der vorstehenden Ansprüche, wobei das Hotmelt-Material ein metallocenkatalysiertes Polymer umfasst, das ein Spitzenmolekulargewicht (Mp) in dem Bereich von 10.000 g/mol bis 130.000 g/mol aufweist, wobei das Spitzenmolekulargewicht gemäß dem hierin offenbarten Spitzenmolekulargewichtsmessverfahren gemessen wird, wobei vorzugsweise das Hotmelt-Material zu wenigstens 50 Gew.-% das metallocenkatalysierte Polymer umfasst.

6. Laminat nach einem der vorstehenden Ansprüche, wobei die Innenseite der Substratschicht und die Innenseite der Faserschicht durch ein Haftmittel (6) gebunden sind, insbesondere ein Haftmittel, umfassend einen Klebrigmacher.

7. Laminat nach einem der vorstehenden Ansprüche, wobei die Faserschicht (28) ferner Spunmelt-Fasern (16) umfasst, die mit den Cellulosefasern vermischt sind.

8. Laminat nach dem vorstehenden Anspruch, wobei die Spunmelt-Fasern schmelzgeblasene Fasern (16) sind, insbesondere schmelzgeblasene Polypropylenfasern.

9. Laminat nach einem der vorstehenden Ansprüche, wobei die Substratschicht (2) eine Vliesschicht ist, wobei vorzugsweise die Vliesschicht ein hydrophiles Mittel umfasst.

10. Laminat nach einem der vorstehenden Ansprüche, wobei das Laminat ferner eine Faserbeschichtungszusammensetzung (32) umfasst, die die Fasern auf der Außenseite der Faserschicht wenigstens teilweise beschichtet und bindet.

11. Verfahren zum Herstellen eines Laminats nach einem der vorstehenden Ansprüche, das Verfahren umfassend die Schritte:
- Bereitstellen einer Substratschicht (2), die eine Innenseite und eine Außenseite aufweist;
- wahlweises Aufbringen eines Haftmittels (6) auf die Innenseite der Substratschicht;
- Bereitstellen eines Stroms aus Cellulosefasern (12);
- Sprühen des Hotmelt-Materials (14) in den Strom aus Cellulosefasern;
- wahlweises Spunmelting von Fasern (16) in den Strom aus Cellulosefasern; und anschließend
- Ausbilden der Faserschicht (28) durch Abscheiden des verbundenen Stroms (27) aus Cellulosefasern, Hotmelt-Material und, falls gegenwärtig, Spunmelt-Fasern auf die Innenseite der Substratschicht.

12. Verfahren zum Herstellen eines Laminats nach dem vorstehenden Anspruch, das Verfahren umfassend die Schritte:
- Sprühen einer Faserbeschichtungszusammensetzung (32) auf die Außenseite der abgeschiedenen Faserschicht, sodass die Faserbeschichtungszusammensetzung die Fasern auf dieser Außenseite der Faserschicht wenigstens teilweise beschichtet und bindet.

13. Verfahren nach dem vorstehenden Anspruch, wobei die Faserbeschichtungszusammensetzung ein Hotmelt-Material aus Polymermaterial ist, das gleich dem oder anders als das Hotmelt-Material nach einem der vorstehenden Ansprüche sein kann.

14. Körperhygieneabsorptionsartikel, umfassend eine Oberschicht (42) auf seiner trägerseitigen Seite, ein Aufnahme-Verteilungssystem (40), einen Absorptionskern (46) und eine Unterschicht (44) auf seiner zum Kleidungsstück zeigenden Seite, wobei das Aufnahme-Verteilungssystem ein Laminat nach einem der Ansprüche 1 bis 10 ist, wobei die Substratschicht (2) in Richtung der Oberschicht ausgerichtet ist und die Faserschicht in Richtung des Absorptionskerns ausgerichtet ist.

15. Körperhygieneabsorptionsartikel nach dem vorstehenden Anspruch, wobei der Absorptionskern eine obere Schicht (50a), eine untere Schicht (50b) und eine Absorptionsschicht (48) zwischen der oberen Schicht und der unteren Schicht umfasst, wobei die Absorptionsschicht von zu 0 % bis 20 % Zellulosefasern nach Gewicht der Absorptionsschicht umfasst.

## Revendications

1. Stratifié (40) comprenant une couche de substrat (2) et une couche fibreuse (28), chaque couche ayant un côté interne et un côté externe, dans lequel le côté interne d'une couche fait face vers le côté interne de l'autre couche,
la couche fibreuse comprenant des fibres de cellulose (12) et des morceaux (17) de forme irrégulière de matériau thermofusible (14), dans lequel ces morceaux de matériau thermofusible lient au moins partiellement les fibres de cellulose ensemble, fournissant ainsi une intégrité à la couche fibreuse.

2. Stratifié (40) selon la revendication 1, dans lequel les morceaux (17) de matériau thermofusible lient au moins partiellement les fibres de cellulose au moins à travers une première épaisseur (13) de la couche fibreuse adjacente au côté interne de la couche fibreuse.

3. Stratifié selon l'une quelconque des revendications précédentes, dans lequel le matériau thermofusible a une viscosité à 170 °C allant de 100 mPa.s à 10 000 mPa.s, telle que mesurée par le procédé de test de viscosité décrit ici.

4. Stratifié selon l'une quelconque des revendications précédentes, dans lequel le matériau thermofusible a un module de conservation (G') à 37 °C allant d'environ 3 MPa à 40 MPa, tel que mesuré par le procédé de test de rhéométrie oscillatoire décrit ici.

5. Stratifié selon l'une quelconque des revendications précédentes, dans lequel le matériau thermofusible comprend un polymère catalysé par métallocène ayant une masse moléculaire maximale (Mp) dans la plage allant de 10 000 g/mol à 130 000 g/mol, dans lequel la masse moléculaire maximale est mesurée selon le procédé de mesure de masse moléculaire maximale décrit ici, de préférence dans lequel le matériau thermofusible comprend au moins 50 % en poids du polymère catalysé par métallocène.

6. Stratifié selon l'une quelconque des revendications précédentes, dans lequel le côté interne de la couche de substrat et le côté interne de la couche fibreuse sont liés par un adhésif (6), en particulier un adhésif comprenant un agent conférant de l'adhésivité.

7. Stratifié selon l'une quelconque des revendications précédentes, dans lequel la couche fibreuse (28) comprend en outre des fibres filées par fusion (16) entremêlées avec les fibres de cellulose.

8. Stratifié selon la revendication précédente, dans lequel les fibres filées par fusion sont des fibres soufflées en fusion (16), en particulier des fibres de polypropylène soufflées en fusion.

9. Stratifié selon l'une quelconque des revendications précédentes, dans lequel la couche de substrat (2) est une couche de non-tissé, de préférence dans lequel la couche de non-tissé comprend un agent hydrophile.

10. Stratifié selon l'une quelconque des revendications précédentes, dans lequel le stratifié comprend en outre une composition de revêtement de fibre (32) qui revêt et lie au moins partiellement les fibres sur le côté externe de la couche fibreuse.

11. Procédé permettant de fabriquer un stratifié selon l'une quelconque des revendications précédentes, le procédé comprenant les étapes consistant à :
- fournir une couche de substrat (2) ayant un côté interne et un côté externe ;
- appliquer facultativement un adhésif (6) sur le côté interne de la couche de substrat ;
- fournir un courant de fibres de cellulose (12) ;
- pulvériser le matériau thermofusible (14) dans le courant de fibres de cellulose ;
- filer facultativement en fusion les fibres (16) dans le courant de fibres de cellulose ; puis
- former la couche fibreuse (28) en déposant le courant combiné (27) de fibres de cellulose, de matériau thermofusible et, si elles sont présentes, de fibres filées par fusion sur le côté interne de la couche de substrat.

12. Procédé permettant de fabriquer un stratifié selon la revendication précédente, le procédé comprenant en outre les étapes consistant à :
- pulvériser une composition de revêtement de fibre (32) sur le côté externe de la couche fibreuse déposée de sorte que la composition de revêtement de fibre revêt et lie au moins partiellement les fibres sur ce côté externe de la couche fibreuse.

13. Procédé selon la revendication précédente, dans lequel la composition de revêtement de fibre est un matériau polymère en matériau thermofusible, qui peut être le même que ou différent du matériau thermofusible tel qu'indiqué dans l'une quelconque des revendications précédentes.

14. Article absorbant d'hygiène personnelle comprenant une feuille de dessus (42) sur son côté faisant face vers le porteur, un système de recueil-répartition (40), une âme absorbante (46) et une feuille de fond (44) sur son côté faisant face vers le vêtement, dans lequel le système de recueil-répartition est un stratifié selon l'une quelconque des revendications 1 à 10, dans lequel la couche de substrat (2) est orientée vers la feuille de dessus et la couche fibreuse est orientée vers l'âme absorbante.

15. Article absorbant d'hygiène personnelle selon la revendication précédente, dans lequel l'âme absorbante comprend une couche supérieure (50a), une couche inférieure (50b) et une couche absorbante (48) entre la couche supérieure et la couche inférieure, dans lequel la couche absorbante comprend de 0 % à 20 % de fibres de cellulose en poids de la couche absorbante.
